# EUROPEAN PATENT SPECIFICATION

(11) **EP 1 687 381 B1**
(45) Date of publication and mention of the grant of the patent: **11.04.2007**
(21) Application number: 04799166.6
(22) Date of filing: 16.11.2004
(51) Int. Cl.: C09D 11/00, B05D 1/28, G03F 7/00, G03F 7/16

(54) **FORMATION OF SELF-ASSEMBLED MONOLAYERS**
HERSTELLUNG VON SELBSTORGANISIERTEN MONOSCHICHTEN
FORMATION DE MONOCOUCHES AUTO-ASSEMBLEES

(30) Priority: 19.11.2003 GB 0326904
(43) Date of publication of application: 09.08.2006
(73) Proprietor: Koninklijke Philips Electronics N.V., 5621 BA Eindhoven (NL)
(72) Inventor: BURDINSKI, Dirk c/o Philips I.P. & Standards, Redhill, Surrey RH1 5HA (GB)
(74) Representative: Damen, Daniel Martijn
(86) International application number: PCT/IB2004/052448
(87) International publication number: WO 2005/049741

(56) References cited:
- US-A- 5 512 131
- US-A1- 2003 010 241
- M.M.E. METWALLY, C.H. AMUNDSON AND T. RICHARDSON: "Anomalous Calibration Curves in Gas Chromatography of High-Boiling Compounds" ANALYTICAL CHEMISTRY, vol. 39, no. 4, 1967, pages 551-552, XP002313709

## Description

This invention relates to the formation of self-assembled monolayers (SAMs). In a preferred embodiment, the invention relates to the formation of SAMs in microcontact printing and, more particularly, b a new class of ink molecules for the formation of SAMs in microcontact printing, and their use therein.

Patterning a metal over a substrate is a common need and important process in modern technology, and is applied, for example, in microelectronics and display manufacturing. This patterning usually requires the vacuum deposition of a metal over the entire surface of a substrate and its selective removal using photolithography and etching techniques.

Microcontact printing is a technique for forming patterns of organic monolayers with micrometer and submicron lateral dimensions. It offers experimental simplicity and flexibility in forming certain types of patterns by printing molecules from a stamp onto a substrate. So far, most of the prior art relies on the remarkable ability of long chain alkanethiols to form self-assembled monolayers on, for example, gold or other metals. These patterns can act as nanometer-thin resists by protecting the supporting metal from corrosion by appropriately formulated etchants, or can allow for the selective placement of fluids on hydrophilic regions of the printed pattern. Patterns of SAMs having lateral dimensions that can be less than 1 micrometer can be formed by printing them on a metal substrate using an elastomeric "stamp". The stamp is fabricated by moulding, for example, a silicone elastomer using a master (or mould) prepared using photolithography or using other techniques such as electron-beam lithography. For an effective transfer of the ink molecules to the substrate, often low modulus polymer materials, such as PDMS (polydimethylsiloxanes) are used for the stamp. There are, however, in principle no restrictions with respect to the stamp material.

US 5512131 describes a method of patterning a material surface in which an elastomeric stamp having a stamping surface is loaded with a SAM forming species having a functional group selected to bind to a particular material, and the stamping surface is placed against a surface of the material and is removed to leave a SAM of the species according to the stamping surface pattern of the stamp. Additional stamping steps may be subsequently effected to produce any of a variety of SAM patterns on the surface. Additionally, portions of the material surface that are not coated with a stamped SAM pattern may be filled in with another SAM-forming species. Alternatively, portions that are not covered by a SAM layer may be etched or plated.

Patterning of a surface is also disclosed in EP-B-0 784 543, which describes a process for producing lithographic features in a substrate layer, comprising the steps of lowering a stamp carrying a reactant onto a substrate, confining the subsequent reaction to the desired pattern, lifting the stamp and removing the debris of the reaction from the substrate. The stamp may carry the pattern to be etched or depressions corresponding to the pattern.

Thus, microcontact printing is a soft lithographic patterning technique that has the inherent potential for the easy, fast and cheap reproduction of structured surfaces and electronic circuits with medium to high resolution.

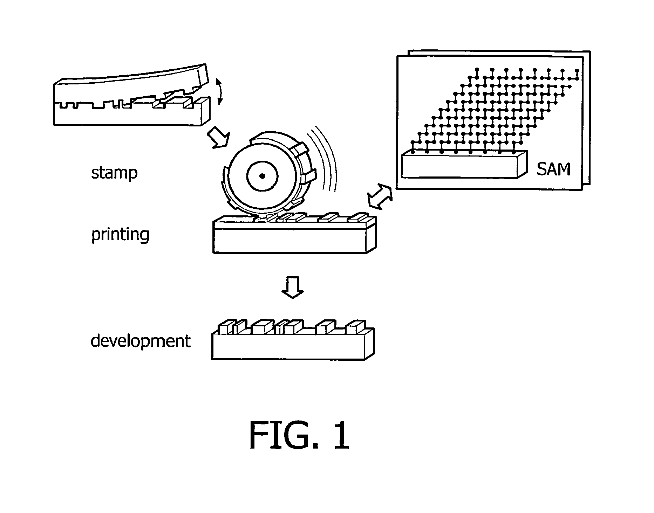

The four main steps of a microcontact process are (with reference to Figure 1 of the drawings):
- Reproduction of a stamp 10 with the desired pattern;
- Loading of the stamp with an appropriate ink solution; and
- Printing with the inked and dried stamp 10 to transfer the pattern 14 from the stamp 10 to the surface 12.

As explained above, printing of higher alkanethiols as ink molecules onto gold and other metal surfaces was one of the first techniques developed for SAMs of deprotonated thiolates on the surface resembling the pattern of the stamp.

The driving force for the formation of the SAM is the strong interaction of the polar thiolate head groups with the gold atoms (or atoms of other metals) in the uppermost surface layer, on the one hand, and the intermolecular (hydrophobic) van der Waals interaction between the apolar tail groups in the SAM, on the other hand. The combination of these two interactions resulted in a well ordered SAM of high stability against mechanical, physical or chemical attack.

It is known that the thiol molecules of ink solutions bind to the metal surface of the substrate during microcontact printing in their deprotonated form, as thiolates:

RSH → RS⁻ + H⁺ (1)

At the same time, oxidation of the gold surface atoms occurs:

[M]_{surface}+ → [M⁽⁺⁾]_{surface}+ e⁻ (2)

to allow formation of a strong bond between a sulphide group and a positively charged gold species in the uppermost metal layer:

RS⁻ + [M⁽⁺⁾]_{surface} → RS⁽⁻⁾ - [M⁽⁺⁾]_{surface} (3)

The oxidizing species that takes up the electron released by the metal surface is the hydrogen ion that disassociates from the alkanethiol (equations 1 and 2):

H⁺ + e⁻ → ½H₂ (↑) (4)

Combination of equations 1 to 4 results in the overall reaction:

RSH + [M]_{surface} → RS⁽⁻⁾ - [M⁽⁺⁾]_{surface} + ½H₂ (↑) (5)

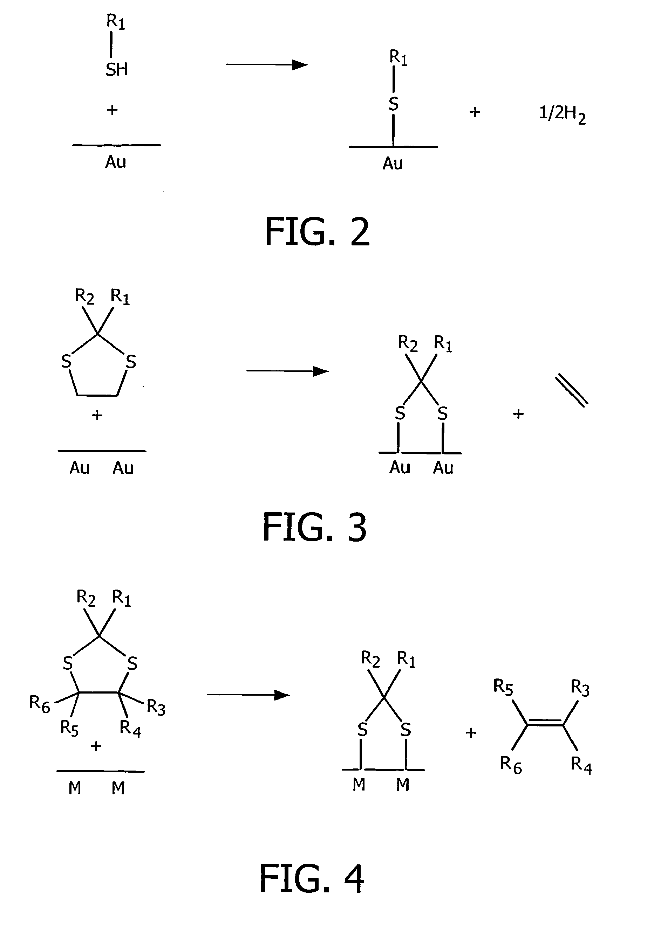

The above prior art reaction scheme is further illustrated in Figure 2.

An identical mechanism can be formulated for other sulphur-functionalised molecular species that have been used as ink molecules for microcontact printing, such as thio- or dithiocarboxylic acids (RCSOH, RCS₂H) and sulfinic acids (RSO₂H), all of them bearing an S-H terminal functional group.

In fact, various ink molecules are in wide-spread use in the field of microcontact printing, such as alkanethiols (RSH), dialkyldisulfides (RSSR), dialkylsulfides (R₂S) and multi-functional alkanethiols (X(-R-SH)*ₙ*, *n*=1-6). Recently proposed ink molecules for printing on noble metal surfaces are 2-mono- and 2,2 di-substituted propane-1,3 dithiols (R¹R²C((CH₂)SH)₂), thiocarboxylic acids (RCOSH) and dithiocarboxylic acids (RCS₂H). For example, International Patent Application No. WO 02/071151 A1 describes a method of microcontact printing in which a dithiocarboxylic acid is used as the ink molecule in the formation of a SAM. In all these cases, it is the formal reduction of protons and the release of dihydrogen that are necessary to counterbalance the surface oxidation during the adsorption process.

One problem, however, in the application of microcontact printing to the patterning of metals is the limited number of suitable classes of ink molecules that are currently available and also the stability of these currently available inks during storage. This allows only limited variability in the development of tailor-made microcontact printing technologies, in particular as low shelf life times of ink solutions can hamper the applicability of these inks.

We have now devised an improved arrangement, and in a particular preferred embodiment the present invention can provide improved ink compositions for microcontact printing, which alleviates the problems associated with prior art ink compositions as discussed above.

There is provided by the present invention, therefore, a method of forming a SAM on at least one surface of a substrate by application to said surface of a 2-mono-, or 2,2-disubstituted 1,3-dithiacyclopentane so as to form a SAM prepared therefrom on said surface.

More particularly, there is provided by the present invention a method of forming a SAM on at least one surface of a substrate by application to said surface of a compound of formula (I) so as to form a SAM prepared therefrom on said surface 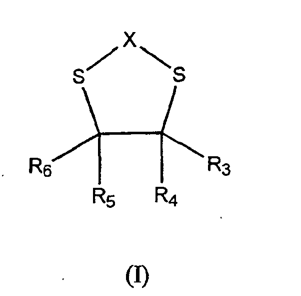 where X can represent either 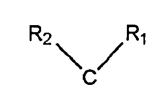 or 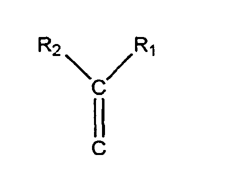 wherein
one of R₁ and R₂ can represent hydrogen and at least one of R₁ and R₂ independently represents a hydrocarbon or halogenated hydrocarbon containing group, optionally provided with a selected functionality that can bind a selected biological or chemical species, or at least one of R₁ and R₂ can comprise a selected biological or chemical species directly or indirectly attached to the 1,3-dithiacyclopentane ring of a compound of formula (I), which selected biological or chemical species is such as to be suitable for immobilization to said surface further to binding of the 1,3-dithiacyclopentane ring, or a derivative thereof, to said surface; and

R₃, R₄, R₅ and R₆, are selected from the group consisting of hydrogen, halogen, -Rₐ, -ORₐ, -SRₐ, -NRₐR_{b}, wherein Rₐ and R_{b} can independently represent hydrocarbon which includes straight chained, branched and cyclic aliphatic and aromatic groups; or (i) R₃ and R₄, and / or (ii) R₅ and R₆, together respectively represent =O.

The term hydrocarbon as used herein can denote straight-chained, branched and cyclic aliphatic and aromatic groups, and can typically include alkyl, alkenyl, alkynyl, cycloalkyl, cycloalkylalkyl, aryl, arylalkyl, arylalkenyl and arylalkynyl. The term "hydrocarbon containing group" also allows for the presence of atoms other than carbon and hydrogen, typically for example, oxygen and / or nitrogen. For example, one or more methylene oxide, or ethylene oxide, moieties may be present in the hydrocarbon containing group; alkylated amino groups may also be useful.

According to a first preferred embodiment of the present invention, X represents 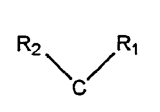 and as such a method according to the present invention employs compounds of formula (1a) 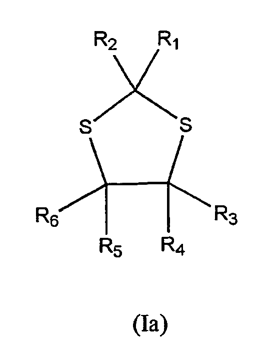 where R₁ to R₆ are substantially as hereinbefore defined.

According to a second preferred embodiment of the present invention, X represents 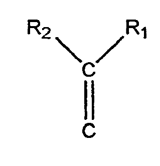 and as such a method according to the present invention employs compounds of formula (lb) 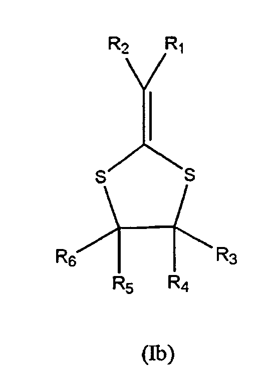 again where R₁ to R₆ are substantially as hereinbefore defined.

Suitably, R₁ represents hydrogen and R₂ represents a hydrocarbon or halogenated hydrocarbon containing group. The term hydrocarbon as explained above can denote straight-chained, branched and cyclic aliphatic and aromatic groups, and can typically include alkyl, alkenyl, alkynyl, cycloalkyl, cycloalkylalkyl, aryl, arylalkyl, arylalkenyl and arylalkynyl. Suitably, the hydrocarbon groups can contain up to 35 carbon atoms, typically up to 30 carbon atoms, and more typically up to 20 carbon atoms. Corresponding halogenated hydrocarbons can also be employed, especially fluorinated hydrocarbons. In a preferred case where R₂ represents a fluorinated alkyl group, this can be represented by the general formula F(CF₂)ₖ(CH₂)ₗ, where k is typically an integer having a value between 1 and 30 and I is an integer having a value of between 0 and 6. More preferably, k is an integer of between 5 and 20, and particularly between 8 and 18. It is of course recognized that although the above are given as preferred ranges for the values of k and I, the particular choice of k and I will depend on the purpose to which the surface to be treated is to be put. It will also be appreciated that the term "hydrocarbon containing group" also allows for the presence of atoms other than carbon and hydrogen, typically O or N, as explained above.

The above hydrocarbon groups can also be further substituted by substituents well known in the art, such as C₁₋₆alkyl, phenyl, C₁₋₆haloalkyl, hydroxy, C₁₋₆alkoxy, C₁₋₆alkoxyalkyl, C₁₋₆alkoxyC₁₋₆alkoxy, aryloxy, keto, C₂₋₆alkoxycarbonyl, C₂₋₆alkoxycarbonylC₁₋₆alkyl, C₂₋₆alkylcarbonyloxy, arylcarbonyloxy, arylcarbonyl, amino, mono- or di- (C₁₋₆)alkylamino, or any other suitable substituents known in the art. In particular, for example in the case where R₁ represent hydrogen and R₂ represents phenyl, this phenyl group can be further substituted by phenylcarbonyloxy where the phenyl group of the above substituent may itself be further substituted by an alkyl group or other suitable substituent.

Preferred dithiacycbpentanes for use according to the present invention can be where R₁ represents hydrogen and R₂ represents alkyl, or aryl, which in turn may be further substituted, for example, by an arylcarbonyloxy substituent as referred to above. In a preferred embodiment, R₁ represents hydrogen and R₂ represents an alkyl group of up to 30 carbon atoms, and more typically up to 20 carbon atoms, and more preferably R₂ represents - (CH₂)₁₆CH₃. In an alternative preferred embodiment, R₁ represent hydrogen and R₂ represents phenyl, which may be further substituted by (alkyl substituted) phenylcarbonyloxy-, and more preferably R₂ can represent the following substituent

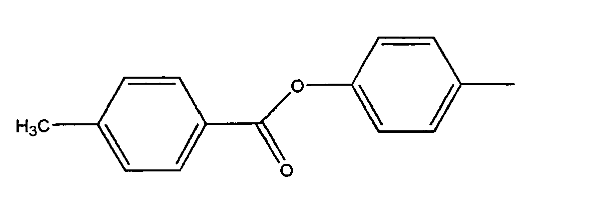

In a further preferred embodiment of the present invention, it can be preferred that R₁ represents hydrogen and R₂ represents a hydrocarbon or halogenated hydrocarbon containing group, provided with a selected functionality that can bind a selected biological or chemical species. Suitably, a selected functionality is provided whereby one or more polymers, dendrimers or biomolecules can be bound by the 1,3-dithiacyclopentanes employed according to the present invention and thus be adsorbed onto the surface of a substrate. In particular, the present invention can allow biomolecules to be adsorbed onto the surface of a metal substrate, such as a coinage metal, such as gold, by binding of such biomolecules to 1,3-dithiacyclopentanes as employed in the present invention. Biomolecules that can be bound to a metal surface according to the present invention can include, for example, proteins, nucleic acids, antibodies, sugars, other carbohydrates and the like. Suitably, one of R₁ or R₂ can be provided with an amino acid functionality so as to facilitate binding of one or more biomolecules to a selected substrate, and suitably one of R₁ or R₂ can represent the following substituent 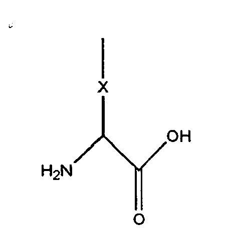 where X can represent a hydrocarbon containing group as hereinbefore described for R₁ or R₂, and more preferably can represent an alkylene linker, such as -(CH₂)ₘ-, where m is typically 1 to 6, or arylene linker, such as - (CH₂)ₙ(P-C₆H₄)(CH₂)ₒ-, where n and o can independently represent an integer of 0 to 3.

Alternatively, at least one of R₁ and R₂ can comprise a selected biological or chemical species directly or indirectly attached to the 1,3-dithiacyclopentane ring of a compound of formula (I), which selected biological or chemical species is such as to be suitable for immobilization to said surface further to binding of the 1,3-dithiacyclopentane ring, or a derivative thereof, to said surface. As such, the dithiacyclopentane functionality is inherent in the structure of the biological or chemical species to be immobilized, with the biological or chemical species either directly attached to the 1,3-dithiacyclopentane ring or indirectly attached thereto, for example by a hydrocarbon or halogenated hydrocarbon containing group substantially as hereinbefore described. For example, a selected peptide or protein could be modified to bear an amino acid as represented by the above formula and as such this could be present as a part of the polypeptide backbone. A "derivative" of the 1,3-dithiacyclopentane ring as referred to above for binding to the substrate surface can typically comprise an intermediate open chain structure obtained further to a redox reaction with the substrate surface (typically a metal), which can be further illustrated by reference to equations (6) and (7) and also Figures 3 to 7.

As referred to above, R₃, R₄, R₅ and R₆, are selected from the group consisting of hydrogen, halogen, -Rₐ, -ORₐ, -SRₐ, -NRₐR_{b}, wherein Rₐ and R_{b} can independently represent a hydrocarbon which includes straight chained, branched and cyclic aliphatic and aromatic groups; or (i) R₃ and R₄, and / or (ii) R₅ and R₆, together respectively represent =O. More suitably, R₃, R₄, R₅ and R₆, are selected from the group consisting of hydrogen, fluoro, chloro, -R_{c}, -OR_{c}, -SR_{c} and -NR_{c}R_{d}, where R_{c} and R_{d} can represent C₁-₆alkyl or C₂₋₆alkenyl. Each of R₃, R₄, R₅ and R₆, can, therefore, represent hydrogen. Alternatively, each of R₃, R₄, R₅ and R₆, can represent halogen, in particular fluoro. A further alternative is where R₃ and R₄ together represent =O, and R₅ and R₆ together represent =O, whereby the compounds for use according to the present invention include 1,3-dithiolane-4,5-diones.

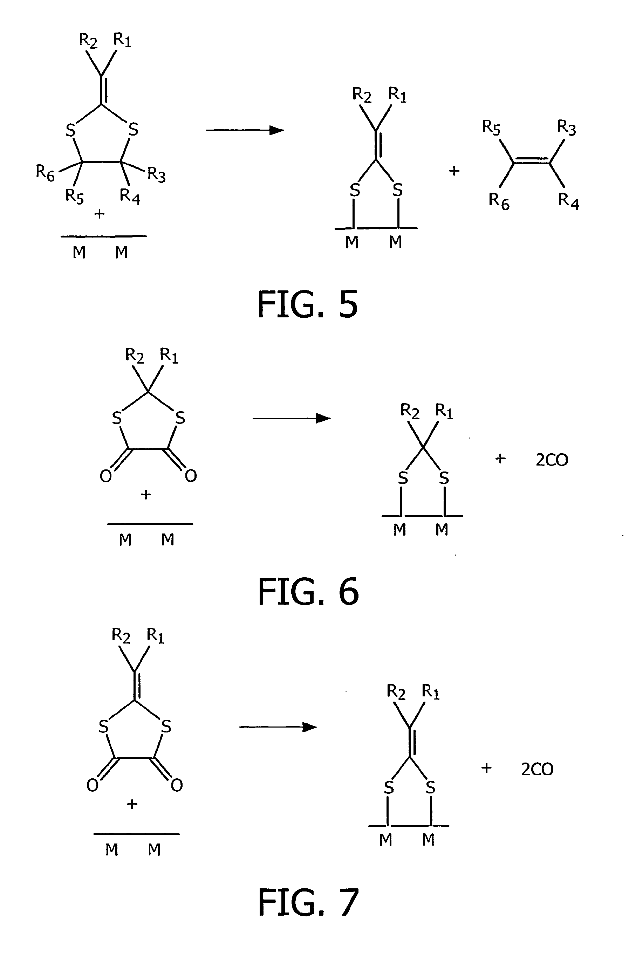

It will be appreciated from the above that various combinations of the above described substituents X, and R₁ to R₆, may be employed in 1,3-dithiacyclopentanes according to the present invention, and as such bind to a metal substrate. For example, for substituents R₁ and R₂, these may be directly bound to the 1,3-dithiacyclopentane ring and binding of such 1,3-dithiacyclopentanes to a metal substrate as achieved by a method according to the present invention can be, for example, as illustrated in Figures 3 and 4. Alternatively, substituents R₁ and R₂ may be bound to the 1,3-dithiacyclopentane ring via an ethenylene linker, as in 1,3-dithiol-2-ylidene derivatives, which can bind to a metal substrate as achieved by a method according to the present invention, for example, as illustrated in Figure 5. The synthesis of such 1,3-dithiol-2-ylidene derivatives has, for instance, been previously described by E. Campaigne et al (Campaigne, E. and F. Haaf, *Dithiolium Derivatives. V. 1,3-Dithiol-2-ylidenes.* Journal of Organic Chemistry, 30, 732-735 (1965)) and Schönberg et al (Schönberg, A., B. König, and E. Frese, *Untersuchungen über die Einwirkung von 4.5-Dioxo-2-thioxo-1.3-dithiolan und Thion-kohlensäureestern auf Diaryl-diazomethane.* Chemische Berichte, 98, 3303-3310 (1965)).

For substituents R₃ to R₆, it may be preferred that each of R₃ to R₆ represents hydrogen substantially as hereinbefore described, as for example, specifically illustrated by Figure 3 and the binding thereof to a metal substrate. A further preferred embodiment, can be where R₃ and R₄ together represent =O, and R₅ and R₆ together represent =O, whereby the compounds for use according to the present invention include 1,3-dithiolane-4,5-diones and binding thereof to a metal substrate is illustrated in Figures 6 and 7, whereby "O=C=C=O" is released which is not stable and will thus decompose into two molecules of carbon monoxide (CO) as shown in Figures 6 and 7. This further decomposition of the "leaving group" into two stable components makes the formation of the SAM highly irreversible and thus contributes to the stability of the SAM. The synthesis of 1,3-dithiolane-4,5-diones as illustrated in Figure 6 is well established in the chemical literature (Jentzsch, J., J. Favian, and R. Mayer, *Einfache Darstellung geminaler Dithiole und einige Folgereaktionen.* Chemische Berichte, 95, 1764-1766 (1962); Bobbio, F.O. and P.A. Bobbio, *Notiz über die Reduktion des Tetrathian- und des Pentathianringes.* Chemische Berichte, 98, 998-1000 (1965); Schauble, J.H., W.A.V. Saun, and J.D. Williams, *Syntheses of Cyclic Bisthioacylals. 1,3-Dithiane-4,6-diones and 1,3-Dithiolane-4,5-dione.* Journal of Organic Chemistry, 39, 2946-2950 (1974)). The synthesis of 1,3-dithiolane-4,5-diones as illustrated in Figure 7 is also well established in the chemical literature *Werkwijze voor het bereiden van fungicide middelen.* Patent The Netherlands NL 6,509,394; Bleisch, S. and R. Mayer, *Die säurekatalysierte, drucklose Umsetzung aliphatischer Ketone und b-Oxo-carbonsäureester mit Schwefelwasserstoff.* Chemische Berichte, 100, 93-100 (1967); Duus, F., *Influence of substituents on preparation and tautomerism of open-chain b-thio keto esters. Structure determination by NMR and infrared spectroscopy.* Tetrahedron, 28(24), 5923-5947 (1972)).

Specific 1,3-dithiacyclopentanes for use in methods according to the present invention include the following

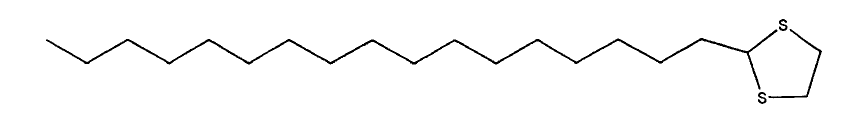

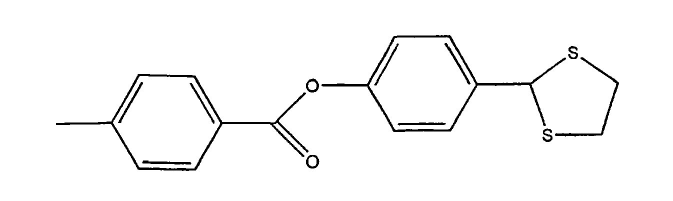

It will be appreciated that certain 2-mono-, or 2,2-disubstituted 1,3-dithiacyclopentanes employed in a method according to the present invention are known compounds. However, certain 2-mono-, or 2,2-disubstituted 1,3-dithiacyclopentanes are novel *per se* and as such form a further aspect of the present invention. More particularly, the present invention further provides a compound of formula (1c) (which is a subgroup of compounds of formula (Ia) 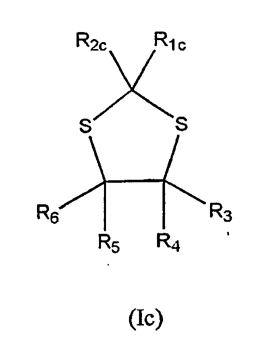 where R_{1c} represents hydrogen, R_{2c} represents C₁₆₋₂₅alkyl and R₃, R₄, R₅ and R₆ are substantially as hereinbefore described. A particularly preferred compounds as provided by the present invention is

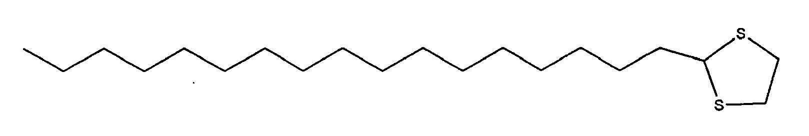

Typically a method according to the present invention can comprise providing a first SAM on a surface of a substrate by application of a 2-mono-, or 2,2-disubstituted 1,3-dithiacyclopentane substantially as hereinbefore described, and further providing a second material to the substrate. The second material can be provided as a SAM selectively formed in areas of the substrate surface substantially uncovered by the first SAM. The 1,3-dithiacyclopentane of the first SAM can be chemically distinct from the molecular species of the second SAM. For example, the first SAM may comprise a hydrophilic monolayer whereas the second SAM may comprise a hydrophobic monolayer. Alternatively, the second material can be a metal or other material, selectively applied to areas of the substrate surface substantially resembling the pattern of the first SAM, with suitable application techniques including electroless deposition of a metal from solution and other suitable techniques known in the art.

SAMs provided according to the present invention can be formed by suitable techniques known in the art, for example by adsorption from solution; or from a gas phase, or may be applied by use of a stamping step employing a flat unstructured stamp or may be applied by a microcontact printing technique which is generally preferred for the provision of at least a first SAM as referred to above.

A preferred embodiment of the invention, therefore, is directed to the provision of a SAM by microcontact printing and there is provided by the present invention, therefore, a method of microcontact printing, comprising printing a pattern on a surface of a substrate, where the pattern includes exposed regions and SAM protected regions, wherein the SAM is formed by application to at least one surface of the substrate of a 2-mono-, or 2,2-disubstituted 1,3-dithiacyclopentane, wherein the substituent at the 2 position facilitates formation of the SAM on the substrate.

Preferably, a method according to the present invention comprises providing a patterned stamp defining the required pattern of said patterned layer; and bringing a patterned stamp loaded with an ink into contact with the surface of said substrate, said patterned stamp being arranged to deliver said ink to the contacted areas of the surface of said substrate; wherein said ink comprises a 2-mono-, or 2,2-disubstituted 1,3-dithiacyclopentane, wherein the substituent at the 2 position facilitates formation of the SAM on the substrate.

The proposed new inks for use in a method according to the present invention have the effect of improving the binding process of sulphur-containing species to metal surfaces. In the case where the leaving molecule is ethylene as illustrated in Figure 3, the reaction scheme can be illustrated further as follows:

R¹R²C(SCH₂)₂ + 2[M]_{surface} → R¹R²C(S⁽⁻⁾ - [M⁽⁺⁾]_{surface})₂ + CH₂ = CH₂ (↑) (6)

In this case, the 1,2-ethylene group inherent in the dithiolane molecule is the oxidising species:

RS-CH₂-CH₂-SR + 2e⁻ → CH₂=CH₂ (↑) + 2RS⁻ (7)

Since the released ethylene product is a less strong reductant than dihydrogen, the oxidation of the metal surface is easier and the formation of a respective monolayer occurs more readily.

1,3-dithiacyclopentanes as employed according to the present invention also provide improved stability of the formed monolayer because the ink molecule may form two possible bonds with the supporting metal surface (see Figures 3 to 7) instead of only one in the case of the simple alkanethiols of the prior art (see Figure 2). In addition, this particular binding arrangement benefits from the stabilising chelate effect in the formed "five-membered ring" at the surface (for example, R¹ R²C(-S-M-)₂).

A further disadvantage associated with the standard alkanethiol ink solutions of the prior art is that such solutions are known to be very unstable against air oxidation due to the oxidation sensitivity of the thiol head groups, causing slow decomposition of these solutions and the formation of insoluble solids.

Once such decomposition has occurred, the solutions are no longer usable. Thus, it is a significant advantage of 1,3-dithiacycbpentanes employed according to the present invention, in that they provide a significantly increased stability against air oxidation.

Typically, a stamp employed in a method according to the present invention includes at least one indentation, or relief pattern, contiguous with a stamping surface defining a first stamping pattern. The stamp can be formed from a polymeric material. Polymeric materials suitable for use in fabrication of a stamp include linear or branched backbones, and may be crosslinked or noncrosslinked, depending on the particular polymer and the degree of formability desired of the stamp. A variety of elastomeric polymeric materials are suitable for such fabrication, especially polymers of the general class of silicone polymers, epoxy polymers and acrylate polymers. Examples of silicone elastomers suitable for use as a stamp include the chlorosilanes. A particularly preferred silicone elastomer is polydimethylsiloxane.

A substrate on which is printed a pattern by use of a method according to the present invention typically comprises a metal substrate, or at least a surface of the substrate on which the pattern is printed comprises a metal, which can suitably be selected from the group consisting of gold, silver, copper, cadmium, zinc, palladium, platinum, mercury, lead, iron, chromium, manganese, tungsten and any alloys of the above. Preferably the substrate, or at least a surface of the substrate on which the pattern is printed, comprises gold.

The present invention also comprises an ink composition for use in microcontact printing, wherein the composition comprises a 2-mono-, or 2,2-disubstituted 1,3-dithiacyclopentane, wherein the substituent at the 2 position facilitates formation of the SAM on a substrate substantially as hereinbefore described, together with a solvent suitable for dissolving the 1,3-dithiacyclopentane.

Suitably, the concentration of 1,3-dithiacyclopentane in a solvent of a composition as provided by the present invention should be selected so as to be low enough that the 1,3-dithiacyclopentane is well-absorbed into a stamping surface of a selected stamp, and high enough that a well defined SAM may be transferred to a substrate surface without blurring. Typically, a 1,3-dithiacyclopentane will be transferred to a stamping surface in a solvent at a concentration of less than 100mM, preferably from about 1.0 to 10.0mM. Any organic solvent within which a 1,3-dithiacyclopentane suitable for use according to the present invention dissolves and which may be absorbed by a stamping surface is suitable. In such selection, if the stamping surface employed is relatively polar, then a relatively polar and / or protic solvent may be advantageously chosen. If a stamping surface employed is relatively non-polar, a relatively non-polar solvent may advantageously be chosen. For example, toluene, ethanol, THF, acetone, isooctane, diethylether and the like may be employed. When a siloxane polymer is selected for fabrication of a stamp for use in a method according to the present invention, and in particular a stamping surface thereof, ethanol is a preferred solvent.

The present invention also provides use of a 2-mono-, or 2,2-disubstituted 1,3-dithiacyclopentane, wherein the substituent at the 2 position facilitates formation of the SAM on a substrate substantially as hereinbefore described, as an ink for use in microcontact printing.

The present invention also provides use of a 2-mono-, or 2,2-disubstituted 1,3-dithiacyclopentane, wherein the substituent at the 2 position facilitates formation of the SAM on a substrate substantially as hereinbefore described, in the manufacture of an ink composition for use in microcontact printing, which use comprises dissolving said 1,3-dithiacyclopentane in a solvent suitable for transferring said 1,3-dithiacyclopentane to a stamping surface. A suitable solvent is substantially as hereinbefore described.

The present invention also provides a method of preparing an ink composition for use in microcontact printing, which method comprises dissolving a 2-mono-, or 2,2-disubstituted 1,3-dithiacyclopentane, wherein the substituent at the 2 position facilitates formation of the SAM on a substrate substantially as hereinbefore described, in a solvent suitable for transferring said 1,3-dithiacyclopentane to a stamping surface. A suitable solvent is again substantially as hereinbefore described.

There is also provided a kit for use in microcontact printing, which kit comprises an ink composition substantially as herein before described; a stamp substantially as hereinbefore described for transferring said 2-mono-, or 2,2-disubstituted 1,3-dithiacyclopentane of said ink composition to a substrate; and a substrate substantially as hereinbefore described suitable for receiving said 2-mono-, or 2,2-disubstituted 1,3-dithiacyclopentane of said ink composition from said stamp.

The present invention still further provides a patterned substrate prepared in accordance with techniques substantially as hereinbefore described. More particularly, the pattern is applied by contacting the substrate with an ink composition comprising a 1,3-dithiacyclopentane substantially as hereinbefore described. The present invention, therefore, provides a substrate provided with a pattern on at least one surface thereof, wherein the pattern includes exposed regions and SAM protected regions, wherein the SAM is formed by application to the surface of a 2-mono-, or 2,2-disubstituted 1,3-dithiacyclopentane, wherein the substituent at the 2 position facilitates formation of the SAM on the substrate substantially as hereinbefore described.

SAM protected regions of a substrate provided according to the present invention desirably exhibit a high stability against etching solutions, and as such exhibit applicability as an etch resist. There is also provided by the present invention a method of etching a substrate, which method comprises providing a SAM to a substrate substantially as hereinbefore described, and subsequently contacting the thus patterned substrate with an etching solution so as to achieve etching in the exposed regions of the substrate not protected by the previously applied SAM. The patterned substrates as provided by the present invention also exhibit applicability in the immobilization of selected chemical and biological materials to the substrate, and as such may also find applicability for use in biochip arrays and biosensors.

1,3-Dithiolanes as employed according to the present invention are typically synthesized through the reaction of carbonyl compounds (aldehydes or ketones) with 1,2-ethanedithiol (1,2-dimercaptoethane) or derivatives thereof, as, for instance, described in US 4,075,228, US 4,096,155, US 4,125,539, or J. March "Advanced Organic Chemistry", 4^{th} Ed., John Wiley & Sons, New York (1992), pp 893-895.

The present invention will now be further illustrated by the following Figures and Experimental, which do not limit the scope of the invention in any way.
Figure 1 is a schematic illustration of the main steps in a method of microcontact printing;
Figure 2 illustrates the reaction of prior art alkanethiols and a gold substrate;
Figure 3 illustrates the reaction of a 1,3-dithiacyclopentane suitable for use in the present invention and a gold substrate, where X represents CR₁R₂ and each of R₃ to R₆ represents hydrogen;
Figure 4 illustrates the reaction of a 1,3-dithiacyclopentane suitable for use in the present invention and a substrate, where X represents CR₁R₂;
Figure 5 illustrates the reaction of a 1,3-dithiacyclopentane suitable for use in the present invention and a substrate, where X represents C=CR₁R₂;
Figure 6 illustrates the reaction of a 1,3-dithiacyclopentane suitable for use in the present invention and a substrate, where X represents CR₁R₂ and R₃ and R₄ together represent =O, and R₅ and R₆ together represent =O;
Figure 7 illustrates the reaction of a 1,3-dithiacyclopentane suitable for use in the present invention and a substrate, where X represents C=CR₁R₂ and R₃ and R₄ together represent =O, and R₅ and R₆ together represent =O;

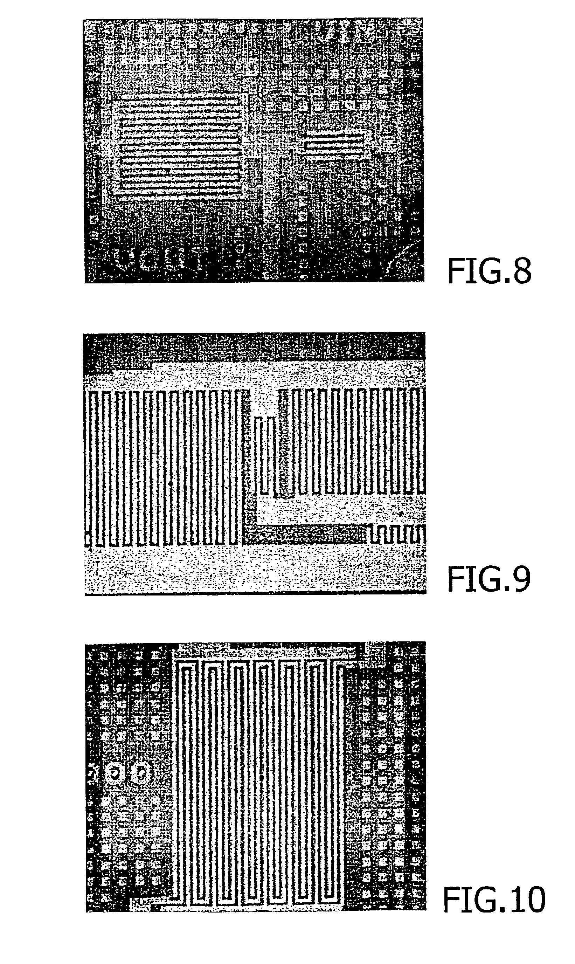

Figure 8 is a microscope photograph of an etched sample obtained during experimentation in respect of an exemplary embodiment of the present invention, in accordance with Example 1;
Figure 9 is a microscope photograph of an etched sample obtained during experimentation in respect of another exemplary embodiment of the present invention, in accordance with Example 2; and
Figure 10 is a microscope photograph of an etched sample obtained during experimentation in respect of yet another exemplary embodiment of the present invention, in accordance with Example 3.

### Experimental

Benzoic acid 4-methyl-4-(1,3-dithiolan-2-yl-)phenyl ester was purchased from the following company: SPECS and BIOSPECS B.V., Fleminglaan 16, Rijswijk 2289 CP, The Netherlands.

### Synthesis of 2-heptadecyl-1,3-dithiolane

Commercially available 1-octadecanol with pyridinium chlorochromate gave 1-octadecanal as described in the following experimental. Treatment with 1,2-ethanedithiol then yielded the desired product, 2-heptadecyl-1,3-dithiolane.

### 1-octadecana I

A lukewarm solution of octadecanol (33.0 g, 0.122 mol) in 300 mL dichloromethane was added over a few minutes to a mixture of pyridinium chlorochromate (40.0 g, 0.186 mol) and 150 mL dichloromethane. The mixture was stirred for 2 h at RT, then 400 mL heptane was added. After stirring for 10 minutes the solution was decanted from the solid and chromatographed on 150g silicagel using the dichloromethane/heptane mixture as the eluent. The colorless eluate was rotary evaporated to give the aldehyde as a solidifying oil. NMR (CDCb): δ 0.9 (t, 3H), 1.2-1.5 (m, 28H), 1.7 (m, 2H), 2.45 (m, 2H), 9.8 (t, 1H).

### 2-heptadecyl-1,3-dithiolane

The aldehyde obtained above was stirred for 2 h with 300 mL dichloromethane, 20 mL 1,2-ethanedithiol, and 2 mL BF₃-etherate. Most of the solvent was removed by rotary evaporation and to the residue there was added heptane containing a little toluene. Chromotography over 100 g silicagel using heptane (with a little toluene) as the eluent gave the crude product which was purified by Kugelrohr distillation (this left a higher boiling impurity behind), followed by recrystallization from 200 mL heptane to give the product (34.04 g, 98.95 mmol, 81 % overall yield). NMR (CDCl₃): δ 0.95 (t, 3H), 1.1-1.6 (m, 30H), 1.9 (m, 2H), 3.3 (m, 4H), 4.55 (t, 1 H).

### Printing Process

### Example 1

Substrates were regular silicium wafers with an about 500 nm thick layer of silicium oxide (thermal oxide). On top of this a 2.5 nm thick layer of titanium was sputtered followed by a 17.5 nm thick gold layer. The uppermost gold surface was rinsed with water, ethanol, and n-heptane and treated with an argon plasma (0.25 mbar, 300 W) for 5 min prior to printing.

A regular poly(dimethylsiloxane) (PDMS) stamp with a size of about 1x2 cm² was used. It was inked with the ink solution at least one hour before printing. This means, that the stamp was immersed in a respective ink solution and stored therein for one hour. The ink solution was a clear and colorless, 2 millimolar solution of 2-heptadecyl-1,3-dithiolane in ethanol. Immediately prior to printing the stamp was taken out of the ink solution and thoroughly rinsed with ethanol to remove all excess ink solution and subsequently dried in a stream of nitrogen for about one minute to remove all ethanol from the surface and from the topmost layer of the stamp material.

The so prepared stamp was brought in contact with the cleaned substrate. Intimate contact over the entire surface was assured by optical inspection. The stamp was removed again after 15 seconds.

Subsequently the printed substrates were developed by wet chemical etching. Thus the monolayer was transferred in the printing step so as to provide a resist, protecting the underlaying gold layer in the printed regions, but allowing undisturbed etching in the not printed regions. Etching was performed by immersing the printed substrates in an etching solution comprising potassium hydroxide (1.0M), potassium thiosulfate (0.1M), potassium ferricyanide (0.01 M), potassium ferrocyanide (0.001 M), water as the solvent and 1-octanol at half saturation at room temperature without special precautions. It was removed after all the gold was etched away in the not protected regions and a clear pattern was visible. The time necessary was about 7 minutes in the indicated etching solution.

### Example 2

A substrate with a top gold layer as described above was prepared for patterning according to the described procedure.

A PDMS stamp was inked and employed for stamping onto the substrate as described in Example 1, except that a 2mM solution of benzoic acid 4-methyl-4-(1,3-dithiolan-2-yl-)phenyl ester in ethanol was used as the ink.

Subsequently the substrate was etched at room temperature in a solution containing potassium hydroxide (1.0M), potassium thiosulfate (0.1M), potassium ferricyanide (0.01 M), and potassium ferrocyanide (0.001 M) for 7 minutes to develop a clear pattern in the gold layer as described in Example1.

### Example 3

A substrate with a top gold layer as described in Example 1 was prepared for patterning according to the described procedure.

A PDMS stamp was inked with a 2mM solution of benzoic acid 4-methyl-4-(1,3-dithiolan-2-yl-)phenyl ester in ethanol and further washed and dried as described in Example 1.

The substrate was printed with the so prepared stamp as described before. Development of the pattern was performed via wet chemical etching in an aqueous etching bath of the following composition: 1.0M thiourea, 0.01 M ferric sulfate, and 0.01 M sulphuric acid. A clear pattern was obtained after an etching time of about 80 seconds at room temperature.

### Example 4

Substrates (1x2cm²) with a composition as described in Example 1 were cleaned according to the procedure described above.

A solution of 2-heptadecyl-1,3-dithiolane in ethanol (2mM) was prepared. The cleaned substrates were immersed in this solution half way, thus one half of the substrates was in contact with the solution and the other half remained outside the solution in the ambient. The substrates were again removed after 30 minutes, washed with ethanol and dried in a stream of nitrogen.

Subsequently the substrates were fully immersed at room temperature in a freshly prepared etching solution containing potassium hydroxide (1.0M), potassium thiosulfate (0.1 M), potassium ferricyanide (0.01 M), potassium ferrocyanide (0.001 M), and n-octanol at half saturation. Samples were again removed from the etching solution after 9, 20, 45, or 80 minutes. In all cases a clear contrast was observed between the two halves of the substrates. The gold layer was completely etched away in the areas that had not been in contact with the dithiolane solution and was virtually unchanged in those areas that had been in contact with the dithiolane solution.

Inspection of the samples by optical microscopy revealed no difference between the sample that had been etched for 10, 20, and 45 minutes. The samples etched for 80 minutes showed some local microscopic defects, indicating a beginning breakdown of the protective self-assembled monolayer.

It should be noted that the above-mentioned embodiments illustrate rather than limit the invention, and that those skilled in the art will be capable of designing many alternative embodiments without departing from the scope of the invention as defined by the appended claims. In the claims, any reference signs placed in parentheses shall not be construed as limiting the claims. The word "comprising" and "comprises", and the like, does not exclude the presence of elements or steps other than those listed in any claim or the specification as a whole. The singular reference of an element does not exclude the plural reference of such elements and vice-versa. The invention may be implemented by means of hardware comprising several distinct elements, and by means of a suitably programmed computer. In a device claim enumerating several means, several of these means may be embodied by one and the same item of hardware. The mere fact that certain measures are recited in mutually different dependent claims does not indicate that a combination of these measures cannot be used to advantage.

## Claims

1. A method of forming a SAM on at least one surface of a substrate by application to said surface of a 2-mono-, or 2,2-disubstituted 1,3-dithiacyclopentane so as to form a SAM prepared therefrom on said surface.

2. A method of forming a SAM on at least one surface of a substrate by application to said surface of a compound of formula (I) so as to form a SAM prepared therefrom on said surface 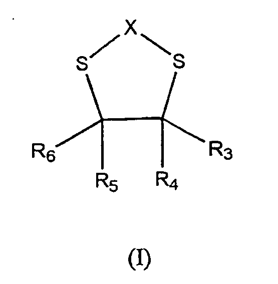 where X can represent either 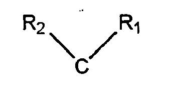 or 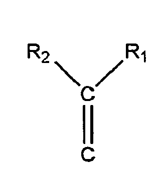 wherein
one of R₁ and R₂ can represent hydrogen and at least one of R₁ and R₂ independently represents a hydrocarbon or halogenated hydrocarbon containing group, optionally provided with a selected functionality that can bind a selected biological or chemical species, or at least one of R₁ and R₂ can comprise a selected biological or chemical species directly or indirectly attached to the 1,3-dithiacyclopentane ring of a compound of formula (I), which selected biological or chemical species is such as to be suitable for immobilization to said surface further to binding of the 1,3-dithiacyclopentane ring, or a derivative thereof, to said surface; and
R₃, R₄, R₅ and R₆, are selected from the group consisting of hydrogen, halogen, -Rₐ, -ORₐ, -SRₐ, -NRₐR_{b}, wherein Rₐ and R_{b} can independently represent hydrocarbon which includes straight chained, branched and cyclic aliphatic and aromatic groups; or (i) R₃ and R₄, and / or (ii) R₅ and R₆, together respectively represent =O.

3. A method according to claim 2, wherein X represents 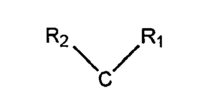 and whereby the SAM is formed by application to at least one surface of the substrate of a compound of formula (1a) 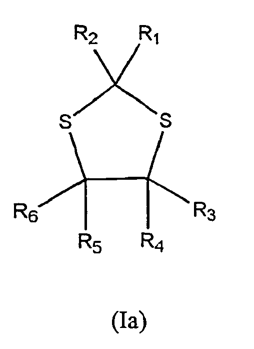 where R₁ to R₆ are as defined in claim 2.

4. A method according to claim 2, wherein X represents 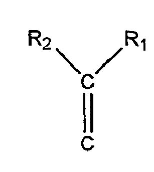 and whereby the SAM is formed by application to at least one surface of the substrate of a compound of formula (Ib) 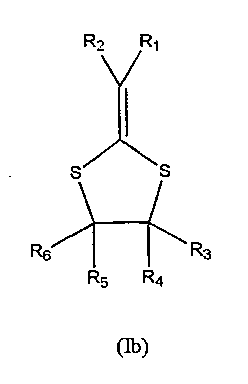 where R₁ to R₆ are as defined in claim 2.

5. A method according to any of claims 2 to 4, wherein R₁ represents hydrogen and R₂ represents a hydrocarbon or halogenated hydrocarbon containing group.

6. A method according to claim 5, wherein R₁ represents hydrogen and R₂ represents alkyl, or aryl, which in turn may be further substituted.

7. A method according to claim 6, wherein R₁ represent hydrogen and R₂ represents an alkyl group of up to 20 carbon atoms.

8. A method according to claim 7, wherein R₂ represents -(CH₂)₁₆CH₃.

9. A method according to claim 6, wherein R₁ represent hydrogen and R₂ represents optionally substituted phenyl.

10. A method according to claim 9, wherein R₂ represents the following substituent

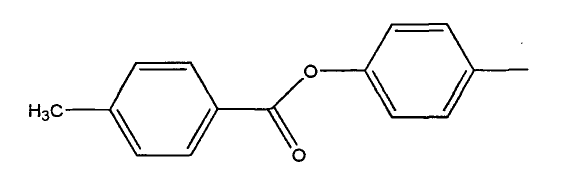

11. A method according to any of claim 2 to 4, wherein R represents hydrogen and R₂ represents a hydrocarbon or halogenated hydrocarbon containing group, provided with said selected functionality that can bind a selected biological or chemical species.

12. A method according to claim 11, wherein said selected functionality allows one or more polymers, dendrimers or biomolecules to be bound by a compound of formula (I).

13. A method according to claim 11 or 12, wherein one of R₁ or R₂ can be provided with an amino acid functionality so as to facilitate binding of one or more biomolecules to a selected substrate.

14. A method according to claim 13, wherein one of R₁ or R₂ can represent the following substituent 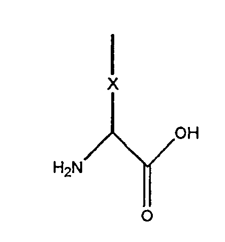 where X can represent a hydrocarbon containing group.

15. A method according to claim 14, wherein X represents either alkylene linker -(CH₂)ₘ-, where m is 1 to 6, or arylene linker -(CH₂)ₙ(p-C₆H₄)(CH₂)ₒ-, where n and o independently represent an integer of 0 to 3.

16. A method according to any of claims 2 to 4, wherein at least one of R₁ and R₂ comprises said selected biological or chemical species directly or indirectly attached to the 1,3-dithiacyclopentane ring of a compound of formula (I).

17. A method according to any of claims 2 to 16, wherein R₃, R₄, R₅ and R₆, are selected from the group consisting of hydrogen, fluoro, chloro, -R_{c}, -OR_{c}, -SR_{c} and-NR_{c}R_{d}, where R_{c} and R_{d} represent C₁₋₆alkyl or C₂₋₆alkenyl.

18. A method according to claim 17, wherein each of R₃, R₄, R₅ and R₆ represent hydrogen.

19. A method according to claim 17, wherein each of R₃, R₄, R₅ and R₆ represent halogen.

20. A method according to claim 19, wherein each of R₃ R₄, R₅ and R₆ represent fluoro.

21. A method according to any of claims 2 to 16, wherein R₃ and R₄ together represent =O, and R₅ and R₆ together represent =O.

22. A method according to any of claims 1 to 21, which further comprises providing a second material to at least one surface of the substrate.

23. A method according to claim 22, wherein the second material is provided as a SAM selectively formed in areas of the substrate surface substantially uncovered by a first SAM formed on said surface according to any of claims 1 to 21.

24. A method according to claim 23, wherein the 1,3-dithiacyclopentane of the first SAM is chemically distinct from the molecular species of the second SAM.

25. A method according to claim 24, wherein the first SAM comprises a hydrophilic monolayer and the second SAM comprises a hydrophobic monolayer.

26. A method according to claim 22, wherein the second material is selectively applied to areas of the substrate surface substantially resembling the pattern of the first SAM formed on said surface according to any of claims 1 to 21.

27. A method according to claim 26, wherein the second material is a metal.

28. A method of microcontact printing, comprising printing a pattern on a surface of a substrate, where the pattern includes exposed regions and SAM protected regions, wherein the SAM is formed by application to at least one surface of the substrate of a 2-mono-, or 2,2-disubstituted 1,3-dithiacyclopentane, wherein the substituent at the 2 position facilitates formation of the SAM on the substrate.

29. A method according to claim 28, wherein said 1,3-dithiacyclopentane is as defined in any of claims 1 to 21.

30. A method according to claim 28 or 29, which comprises providing a patterned stamp defining the required pattern of said patterned layer; and bringing said patterned stamp loaded with an ink into contact with the surface of said substrate, said patterned stamp being arranged to deliver said ink to the contacted areas of the surface of said substrate; wherein said ink comprises said 2-mono-, or 2,2-disubstituted 1,3-dithiacyclopentane.

31. A method according to claim 30, wherein the stamp is formed from polydimethylsiloxane.

32. A method according to any of claims 1 to 31, wherein the substrate comprises a metal substrate, or at least a surface of the substrate on which said SAM is formed comprises a metal.

33. A method according to claim 32, wherein the metal is gold.

34. A method of microcontact printing, comprising printing a pattern on a surface of a substrate, where the pattern includes exposed regions and SAM protected regions, wherein the SAM is formed by application to at least one surface of the substrate of the following 2-monosubstituted 1,3-dithiacyclopentane

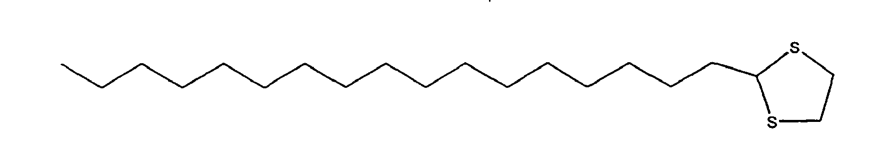

35. A method of microcontact printing, comprising printing a pattern on a surface of a substrate, where the pattern includes exposed regions and SAM protected regions, wherein the SAM is formed by application to at least one surface of the substrate of the following 2-monosubstituted 1,3-dithiacyclopentane

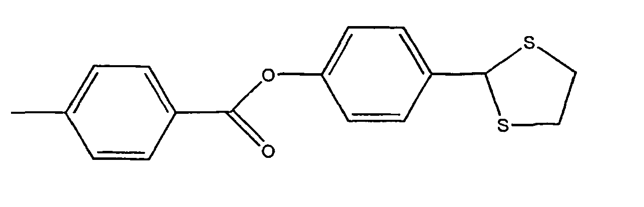

36. An ink composition for use in microcontact printing, wherein the composition comprises a 2-mono-, or 2,2-disubstituted 1,3-dithiacyclopentane, wherein the substituent at the 2 position facilitates formation of the SAM on a substrate, together with a solvent suitable for dissolving the 1,3-dithiacyclopentane.

37. An ink composition according to claim 36, wherein said 2-mono-, or 2,2-disubstituted 1,3-dithiacyclopentane is as further defined in any of claims 1 to 21.

38. An ink composition according to claim 36 or 37, wherein the concentration of said 1,3-dithiacyclopentane in said solvent is less than 100mM.

39. An ink composition according to claim 38, wherein the concentration of said 1,3-dithiacyclopentane in said solvent is in the range of about 1.0 to 10.0mM.

40. An ink composition according to any of claims 36 to 39, wherein said solvent is ethanol.

41. A compound of formula (Ic) 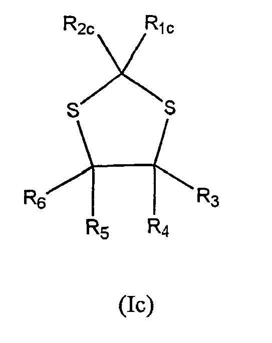 where R_{1c} represents hydrogen, R_{2c} represents C₁₆₋₂₅alkyl and R₃, R₄, R₅ and R₆ are as defined in any of claims 2 and 17 to 21.

42. A compound as claimed in Claim 41, wherein R_{2c} represents a heptadecyl a nd wherein R₃, R₄, R₅ and R₆ represent hydrogen.

43. Use of a 2-mono-, or 2,2-disubstituted 1,3-dithiacyclopentane, wherein the substituent at the 2 position facilitates formation of the SAM on a substrate, as an ink for use in microcontact printing.

44. Use according to claim 43, wherein said 2-mono-, or 2,2-disubstituted 1,3-dithiacyclopentane is as further defined in any of claims 1 to 21.

45. Use of a 2-mono-, or 2,2-disubstituted 1,3-dithiacyclopentane, wherein the substituent at the 2 position facilitates formation of the SAM on a substrate, in the manufacture of an ink composition for use in microcontact printing, which use comprises dissolving said 1,3-dithiacyclopentane in a solvent suitable for transferring said 1,3-dithiacyclopentane to a stamping surface.

46. Use according to claim 45, wherein said 2-mono-, or 2,2-disubstituted 1,3-dithiacyclopentane is as further defined in any of claims 1 to 21.

47. Use according to claim 45 or 46, wherein said solvent is ethanol.

48. A method of preparing an ink composition for use in microcontact printing, which method comprises dissolving a 2-mono-, or 2,2-disubstituted 1,3-dithiacyclopentane, wherein the substituent at the 2 position facilitates formation of the SAM on a substrate, in a solvent suitable for transferring said 1,3-dithiacyclopentane to a stamping surface.

49. A method according to claim 48, wherein said 2-mono-, or 2,2-disubstituted 1,3-dithiacyclopentane is as further defined in any of claims 1 to 21.

50. A method according to claim 48 or 49, wherein said solvent is ethanol.

51. A kit for use in microcontact printing, which kit comprises an ink composition according to any of claims 36 to 40; a microcontact printing stamp for transferring said 2-mono-, or 2,2-disubstituted 1,3-dithiacyclopentane of said ink composition to a substrate; and a substrate suitable for receiving said 2-mono-, or 2,2-disubstituted 1,3-dithiacyclopentane of said ink composition from said stamp.

52. A patterned substrate prepared in accordance with a method according to any of claims 1 to 35.

53. A substrate provided with a pattern on at least one surface thereof, wherein the pattern includes exposed regions and SAM protected regions, wherein the SAM is formed by application to the surface of a 2-mono-, or 2,2-disubstituted 1,3-dithiacyclopentane, wherein the substituent at the 2 position facilitates formation of the SAM on the substrate.

54. A substrate according to claim 53, wherein said 2-mono-, or 2,2-disubstituted 1,3-dithiacyclopentane is as further defined in any of claims 1 to 21.

55. Use of a substrate according to any of claims 52 to 54, as an etch resist.

56. A method of etching a substrate, which method comprises providing a SAM to a substrate according to any of claims 1 to 35, and subsequently contacting the thus patterned substrate with an etching solution so as to achieve etching in the exposed regions of the substrate substantially not protected by the previously applied SAM.

57. Use of a substrate according to any of claims 52 to 54, in the immobilization of selected chemical and biological materials thereto.

58. Use of a 2-mono-, or 2,2-disubstituted 1,3-dithiacyclopentane in the immobilization of selected chemical and biological materials to at least one surface of a substrate.

59. Use according to claim 57 or 58, wherein said biological species is selected form the group consisting of peptides, proteins, oligo- and polynucleic acids.

60. Use according to claim 57 or 58, wherein said chemical species is a polymer or dendrimer.

## Patentansprüche

1. Verfahren zur Bildung einer SAM auf mindestens einer Oberfläche eines Substrats durch Aufbringung auf die Oberfläche eines 2-mono- oder 2,2-disubstituierten 1,3-Dithiacyclopentans, um auf der Oberfläche eine daraus hergestellte SAM zu bilden.

2. Verfahren zur Bildung einer SAM auf mindestens einer Oberfläche eines Substrats durch Aufbringung auf die Oberfläche einer Verbindung nach Formel (I), um auf der Oberfläche eine daraus hergestellte SAM zu bilden 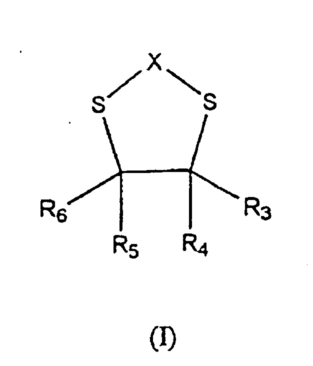 worin X entweder für 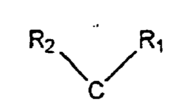 oder für 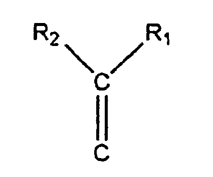 stehen kann, worin
eines von R₁ und R₂ für Wasserstoff stehen kann und mindestens eines von R₁ und R₂ unabhängig voneinander für einen Kohlenwasserstoff oder eine halogenierte kohlenwasserstoffhaltige Gruppe stehen kann, die wahlweise mit einer ausgewählten Funktionalität bereitgestellt ist, die eine biologische oder chemische Spezies binden kann, oder mindestens eines von R₁ und R₂ eine ausgewählte biologische oder chemische Spezies umfassen kann, die direkt oder indirekt an den 1,3-Dithiacyclopentanring einer Verbindung gemäß Formel (I) gebunden ist, wobei die biologische oder chemische Spezies derart ist, dass sie zur Immobilisierung auf der Oberfläche und zur Bindung des 1,3-Dithiacyclopentanrings oder eines Derivats davon an die Oberfläche geeignet ist; und
R₃, R₄, R₅ und R₆, ausgewählt sind aus der Gruppe, bestehend aus Wasserstoff, Halogen, -Rₐ, -ORₐ, -SRₐ, -NRₐR_{b}, worin Rₐ und R_{b} unabhängig voneinander für Kohlenwasserstoff stehen, der geradkettige, verzweigte und zyklische aliphatische und aromatische Gruppen aufweist; oder (i) R₃ und R₄, und/oder (ii) R₅ und R₆, zusammen jeweils für = O stehen.

3. Verfahren nach Anspruch 2, worin X für 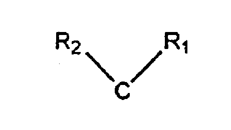 steht und wobei die SAM durch Aufbringung auf mindestens eine Oberfläche des Substrats einer Verbindung gemäß Formel (Ia) gebildet wird 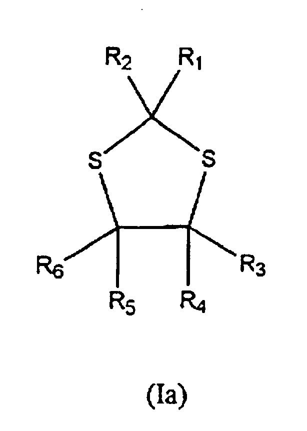 worin R₁ bis R₆ wie in Anspruch 2 definiert sind.

4. Verfahren nach Anspruch 2, worin X für 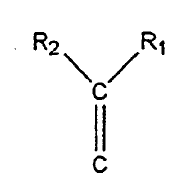 steht und wobei die SAM durch Aufbringung auf mindestens eine Oberfläche des Substrats einer Verbindung gemäß Formel (Ib) gebildet wird 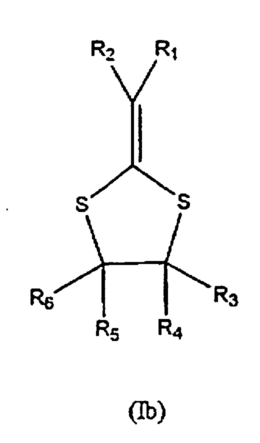 worin R₁ bis R₆ wie in Anspruch 2 definiert sind.

5. Verfahren nach einem der Ansprüche 2 bis 4, worin R₁ für Wasserstoff und R₂ für einen Kohlenwasserstoff oder eine halogenierte kohlenwasserstoffhaltige Gruppe steht.

6. Verfahren nach Anspruch 5, worin R₁ für Wasserstoff und R₂ für Alkyl oder Aryl steht, das wiederum weiter substituiert werden kann.

7. Verfahren nach Anspruch 6, worin R₁ für Wasserstoff und R₂ für eine Alkylgruppe von bis zu 20 Kohlenstoffatomen steht.

8. Verfahren nach Anspruch 7, worin R₂ für-(CH₂)₁₆CH₃ steht.

9. Verfahren nach Anspruch 6, worin R₁ für Wasserstoff steht und R₂ wahlweise für substituiertes Phenyl steht

10. Verfahren nach Anspruch 9, worin R₂ für den folgenden Substituenten steht

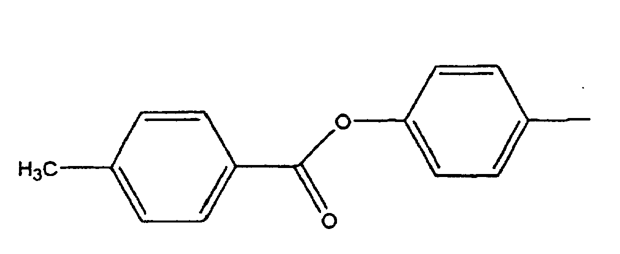

11. Verfahren nach einem der Ansprüche 2 bis 4, worin R₁ für Wasserstoff steht und R₂ für einen Kohlenwasserstoff oder eine halogenierte kohlenwasserstoffhaltige Gruppe steht, die mit der ausgewählten Funktionalität bereitgestellt ist, welche eine ausgewählte biologische oder chemische Spezies binden kann.

12. Verfahren nach Anspruch 11, wobei die ausgewählte Funktionalität ermöglicht, dass ein oder mehrere Polymere, Dendrimere oder Biomoleküle durch eine Verbindung gemäß Formel (I) gebunden werden.

13. Verfahren nach Anspruch 11 oder 12, worin eines von R₁ oder R₂ mit einer Aminosäurefunktionalität bereitgestellt werden kann, um die Bindung eines oder mehrerer Biomoleküle an ein ausgewähltes Substrat zu ermöglichen.

14. Verfahren nach Anspruch 13, worin eines von R₁ oder R₂ für den folgenden Substituenten stehen kann 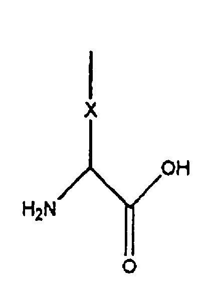 worin X für eine kohlenwasserstoffhaltige Gruppe steht.

15. Verfahren nach Anspruch 14, worin X für entweder einen Alkylenlinker - (CH₂)ₘ₋, worin m 1 bis 6 ist, oder für einen Arylenlinker -(CH₂)ₙ(p-C₆H₄)(CH₂)ₒ₋ steht, worin n und o unabhängig voneinander für eine ganze Zahl von 0 bis 3 stehen.

16. Verfahren nach einem der Ansprüche 2 bis 4, worin mindestens eines von R₁ und R₂ die ausgewählte biologische oder chemische Spezies umfasst, die direkt oder indirekt an den 1,3-Dithiacyclopentanring einer Verbindung gemäß Formel (I) gebunden ist.

17. Verfahren nach einem der Ansprüche 2 bis 16, worin R₃, R₄, R₅ und R₆ ausgewählt sind aus der Gruppe, bestehend aus Wasserstoff, Fluor, Chlor, -R_{c}, -OR_{c}, -SR_{c} und -NR_{c}R_{d}, worin R_{c} und R_{d} für C₁₋₆-Alkyl oder C₂-₆-Alkenyl stehen.

18. Verfahren nach Anspruch 17, worin jedes von R₃, R₄, R₅ und R₆ für Wasserstoff steht.

19. Verfahren nach Anspruch 17, worin jedes von R₃, R₄, R₅ und R₆ für Halogen steht.

20. Verfahren nach Anspruch 19, worin jedes von R₃, R₄, R₅ und R₆ für Fluor steht.

21. Verfahren nach einem der Ansprüche 2 bis 16, worin R₃ und R₄ zusammen für =O stehen und R₅ und R₆ zusammen für =O stehen.

22. Verfahren nach einem der Ansprüche 1 bis 21, ferner umfassend das Bereitstellen eines zweiten Materials mindestens einer Oberfläche des Substrats.

23. Verfahren nach Anspruch 22, wobei das zweite Material als eine SAM bereitgestellt wird, die in Bereichen der Substratoberfläche selektiv gebildet wird, die von einer ersten SAM, die auf der Oberfläche nach einem der Ansprüche 1 bis 21 gebildet wird, im Wesentlichen unbedeckt sind.

24. Verfahren nach Anspruch 23, wobei sich das 1,3-Dithiacyclopentan der ersten SAM von der molekularen Spezies der zweiten SAM chemisch unterscheidet.

25. Verfahren nach Anspruch 24, wobei die erste SAM eine hydrophile Monoschicht und die zweite SAM eine hydrophobe Monoschicht umfasst.

26. Verfahren nach Anspruch 22, wobei das zweite Material auf Bereiche der Substratoberfläche selektiv aufgebracht wird, welche dem Muster der ersten SAM, die auf der Oberfläche nach einem der Ansprüche 1 bis 21 gebildet wird, im Wesentlichen ähnlich sind.

27. Verfahren nach Anspruch 26, wobei das zweite Material ein Metall ist.

28. Verfahren zum Mikrokontaktdrucken, umfassend das Drucken eines Musters auf eine Oberfläche eines Substrats, wobei das Muster freiliegende Bereiche und die SAM geschützte Bereiche aufweist, wobei die SAM durch Aufbringung auf mindestens eine Oberfläche des Substrats eines 2-mono- oder 2,2-disubstituierten 1,3-Dithiacyclopentans gebildet wird, wobei der Substituent an Position 2 die Bildung der SAM auf dem Substrat ermöglicht.

29. Verfahren nach Anspruch 28, wobei das 1,3-Dithiacyclopentan wie in einem der Ansprüche 1 bis 21 definiert ist.

30. Verfahren nach Anspruch 28 oder 29, umfassend das Bereitstellen eines gemusterten Stempels, der das erforderte Muster der gemusterten Schicht definiert; und das Inkontaktbringen des gemusterten Stempels, der mit einer Tinte beladen ist, mit der Oberfläche des Substrats, wobei der gemusterte Stempel angeordnet ist, um die Tinte zu den in Kontakt stehenden Bereichen der Oberfläche des Substrats zu leiten; wobei die Tinte das 2-mono- oder 2,2-disubstituierte 1,3-Dithiacyclopentan umfasst.

31. Verfahren nach Anspruch 30, wobei der Stempel aus Polydimethylsiloxan gebildet wird.

32. Verfahren nach einem der Ansprüche 1 bis 31, wobei das Substrat ein Metallsubstrat umfasst oder mindestens eine Oberfläche des Substrats, auf welchem die SAM gebildet wird, ein Metall umfasst.

33. Verfahren nach Anspruch 32, wobei das Metall Gold ist.

34. Verfahren zum Mikrokontaktdrucken, umfassend das Drucken eines Musters auf eine Oberfläche eines Substrats, wobei das Muster freiliegende Bereiche und die SAM geschützte Bereiche aufweist, wobei die SAM durch Aufbringung auf mindestens eine Oberfläche des Substrat des folgenden 2-monosubstituierten 1,3-Dithiacyclopentans gebildet wird

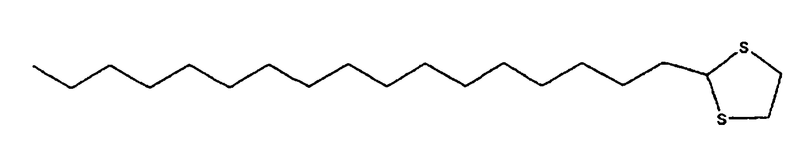

35. Verfahren zum Mikrokontaktdrucken, umfassend das Drucken eines Musters auf eine Oberfläche eines Substrats, wobei das Muster freiliegende Bereiche und die SAM geschützte Bereiche aufweist, wobei die SAM durch Aufbringung auf mindestens eine Oberfläche des Substrat des folgenden 2-monosubstituierten 1,3-Dithiacyclopentans gebildet wird

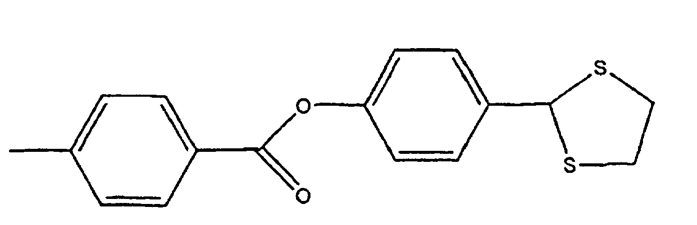

36. Tintenzusammensetzung zur Verwendung beim Mikrokontaktdrucken, wobei die Zusammensetzung ein 2-mono- oder 2,2-disubstituiertes 1,3-Dithiacyciopentan umfasst, wobei der Substituent an Position 2 die Bildung der SAM auf einem Substrat zusammen mit einem Lösungsmittel ermöglicht, das zum Auflösen des 1,3-Dithiacyclopentans geeignet ist.

37. Tintenzusammensetzung nach Anspruch 36, wobei das 2-mono- oder 2,2-disubstituierte 1,3-Dithiacyclopentan wie weiter in einem der Ansprüche 1 bis 21 definiert ist.

38. Tintenzusammensetzung nach Anspruch 36 oder 37, wobei die Konzentration des 1,3-Dithiacyclopentans in dem Lösungsmittel weniger als 100 mM beträgt.

39. Tintenzusammensetzung nach Anspruch 38, wobei die Konzentration des 1,3-Dithiacyclopentans in dem Lösungsmittel im Bereich von etwa 1,0 bis 10,0 mM liegt.

40. Tintenzusammensetzung nach einem der Ansprüche 36 bis 39, wobei das Lösungsmittel Ethanol ist.

41. Verbindung gemäß Formel (Ic) 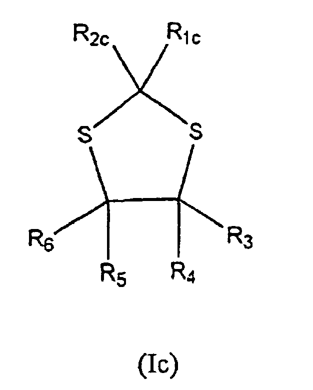 worin R_{1c} für Wasserstoffsteht, R_{2c} für C₁₆₋₂₅-Alkyl steht und R₃, R₄, R₅ und R₆ wie in einem der Ansprüche 2 und 17 bis 21 definiert sind.

42. Verbindung nach Anspruch 41, worin R_{2c} für ein Heptadecyl steht und worin R₃, R₄, R₅ und R₆ für Wasserstoff stehen.

43. Verwendung eines 2-mono- oder 2,2-disubstituierten 1,3-Dithiacyclopentans, wobei der Substituent an Position 2 die Bildung der SAM auf einem Substrat als eine Tinte zur Verwendung beim Mikrokontaktdrucken ermöglicht.

44. Verwendung nach Anspruch 43, wobei das 2-mono- oder 2,2-disubstituierte 1,3-Dithiacyclopentan wie weiter in einem der Ansprüche 1 bis 21 definiert ist.

45. Verwendung eines 2-mono- oder 2,2-disubstituierten 1,3-Dithiacyclopentans, wobei der Substituent an Position 2 bei der Herstellung einer Tintenzusammensetzung zur Verwendung beim Mikrokontaktdrucken die Bildung der SAM auf einem Substrat ermöglicht, wobei diese Verwendung das Auflösen des 1,3-Dithiacyclopentans in einem Lösungsmittel umfasst, das zum Übertragen des 1,3-Dithiacyclopentans auf eine Stempeloberfläche geeignet ist.

46. Verwendung nach Anspruch 45, wobei das 2-mono- oder 2,2-disubstituierte 1,3-Dithiacyclopentan wie weiter in einem der Ansprüche 1 bis 21 definiert ist.

47. Verwendung nach Anspruch 45 oder 46, wobei das Lösungsmittel Ethanol ist.

48. Verfahren zur Herstellung einer Tintenzusammensetzung zur Verwendung beim Mikrokontaktdrucken, wobei das Verfahren das Auflösen eines 2-mono- oder 2,2-disubstituierten 1,3-Dithiacyclopentans, wobei der Substituent an Position 2 die Bildung der SAM auf einem Substrat ermöglicht, in einem Lösungsmittel umfasst, das zum Übertragen des 1,3-Dithiacyclopentans auf eine Stempeloberfläche geeignet ist.

49. Verfahren nach Anspruch 48, wobei das 2-mono- oder 2,2-disubstituierte 1,3-Dithiacyclopentan wie weiter in einem der Ansprüche 1 bis 21 definiert ist.

50. Verwendung nach Anspruch 48 oder 49, wobei das Lösungsmittel Ethanol ist.

51. Satz zur Verwendung beim Mikrokontaktdrucken, wobei der Satz eine Tintenzusammensetzung nach einem der Ansprüche 36 bis 40; einen Mikrokontakt-Druckstempel zum Übertragen des 2-mono- oder 2,2-disubstituierten 1,3-Dithiacyclopentans der Tintenzusammensetzung auf ein Substrat; und ein Substrat umfasst, das zum Aufnehmen des 2-mono- oder 2,2-disubstituierten 1,3-Dithiacyclopentans der Tintenzusammensetzung aus dem Stempel geeignet ist.

52. Gemustertes Substrat, das gemäß einem Verfahren nach einem der Ansprüche 1 bis 35 hergestellt wird.

53. Substrat, das mit einem Muster auf mindestens einer Oberfläche davon bereitgestellt wird, wobei das Muster freiliegende Bereiche und die SAM geschützte Bereiche aufweist, wobei die SAM durch Aufbringung auf die Oberfläche eines 2-mono- oder 2,2-disubstituierten 1,3-Dithiacyclopentans gebildet wird, wobei der Substituent an Position 2 die Bildung der SAM auf dem Substrat ermöglicht.

54. Substrat nach Anspruch 53, wobei das 2-mono- oder 2,2-disubstituierte 1,3-Dithiacyclopentan wie weiter in einem der Ansprüche 1 bis 21 definiert ist.

55. Verwendung eines Substrats nach einem der Ansprüche 52 bis 54 als ein Ätzresist.

56. Verfahren zum Ätzen eines Substrats, wobei das Verfahren das Bereitstellen einer SAM einem Substrat nach einem der Ansprüche 1 bis 35 und das nachfolgende Inkontaktbringen des so gemusterten Substrats mit einer Ätzlösung zum Erreichen einer Ätzung in den freiliegenden Bereichen des Substrats umfasst, die von der vorher aufgebrachten SAM im Wesentlichen nicht geschützt sind.

57. Verwendung eines Substrats nach einem der Ansprüche 52 bis 54 bei der Immobilisierung ausgewählter chemischer und biologischer Materialien darauf.

58. Verwendung eines 2-mono- oder 2,2-disubstituierten 1,3-Dithiacyclopentans bei der Immobilisierung ausgewählter chemischer und biologischer Materialien auf mindestens einer Oberfläche eines Substrats.

59. Verwendung nach Anspruch 57 oder 58, wobei die biologische Spezies ausgewählt ist aus der Gruppe, bestehend aus Peptiden, Proteinen, Oligo- und Polynukleinsäuren.

60. Verwendung nach Anspruch 57 oder 58, wobei die chemische Spezies ein Polymer oder Dendrimer ist.

## Revendications

1. Procédé de formation d'une SAM sur au moins une surface d'un substrat par application à ladite surface d'un 1,3-dithiacyclopentane 2-mono- ou 2,2-disubstitué de manière à former une SAM préparée à partir de celui-ci sur ladite surface.

2. Procédé de formation d'une SAM sur au moins une surface d'un substrat par application à ladite surface d'un composé de formule (I) de manière à former une SAM préparée à partir de celui-ci sur ladite surface : 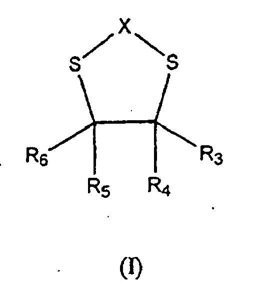 où X peut représenter : 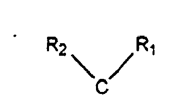 ou 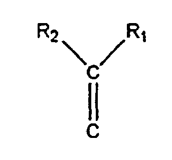 dans laquelle :
l'un des groupements R₁ et R₂ peut représenter de l'hydrogène et au moins un des groupements R₁ et R₂ représente indépendamment un groupement contenant des hydrocarbures ou des hydrocarbures halogénés, pourvu éventuellement d'une fonctionnalité choisie qui peut se lier à une substance biologique ou chimique choisie, ou au moins l'un des groupements R₁ et R₂ peut comprendre une substance biologique ou chimique choisie directement ou indirectement fixée au cycle de 1,3-dithiacyclopentane d'un composé de formule (I), laquelle substance biologique ou chimique choisie est telle qu'elle convienne à une immobilisation sur ladite surface en se liant encore au cycle de 1,3-dithiacyclopentane, ou à un de ses dérivés, sur ladite surface; et
R₃, R₄, R₅ et R₆ sont choisis dans le groupe constitué de l'hydrogène, d'un halogène, de -Rₐ, -ORₐ, -SRₐ, -NRₐR_{b}, où Rₐ et R_{b} peuvent représenter indépendamment un hydrocarbure, qui comprend des groupements aliphatiques et aromatiques à chaîne droite, ramifiés et
cycliques; ou (i) R₃ et R₄ et/ou (ii) R₅ et R₆ représentent conjointement respectivement =O.

3. Procédé selon la revendication 2, dans lequel X représente : 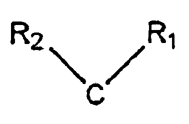 et la SAM est formée par application à au moins une surface du substrat d'un composé de formule (Ia) : 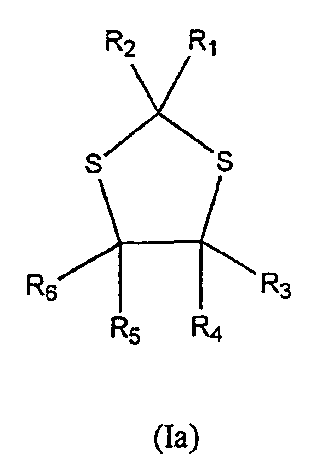 dans laquelle R₁ à R₆ sont comme défini dans la revendication 2.

4. Procédé selon la revendication 2, dans lequel X représente : 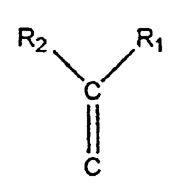 et la SAM est formée par application à au moins une surface du substrat d'un composé de formule (Ib) : 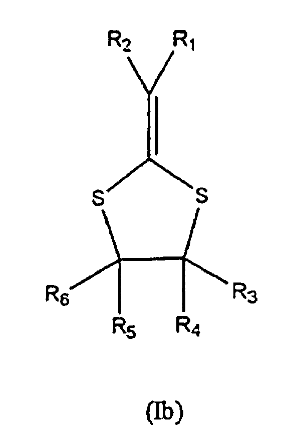 dans laquelle R₁ à R₆ sont comme défini dans la revendication 2.

5. Procédé selon l'une quelconque des revendications 2 à 4, dans lequel R₁ représente de l'hydrogène et R₂ représente un groupement contenant des hydrocarbures ou des hydrocarbures halogénés.

6. Procédé selon la revendication 5, dans lequel R₁ représente de l'hydrogène et R₂ représente un groupement alkyle ou aryle qui, à son tour, peut être encore substitué.

7. Procédé selon la revendication 6, dans lequel R₁ représente de l'hydrogène et R₂ représente un groupement alkyle allant jusqu'à 20 atomes de carbone.

8. Procédé selon la revendication 7, dans lequel R₂ représente -(CH₂)₁₆CH₃.

9. Procédé selon la revendication 6, dans lequel R₁ représente de l'hydrogène et R₂ représente un groupement phényle éventuellement substitué.

10. Procédé selon la revendication 9, dans lequel R₂ représente le substituant suivant :

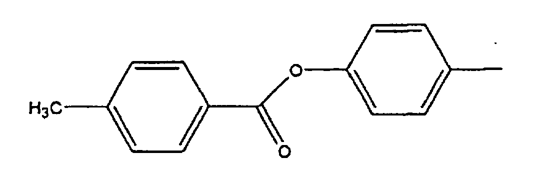

11. Procédé selon l'une quelconque des revendications 2 à 4, dans lequel R₁ représente de l'hydrogène et R₂ représente un groupement contenant des hydrocarbures ou des hydrocarbures halogénés, pourvu de ladite fonctionnalité choisie qui peut se lier à une substance biologique ou chimique choisie.

12. Procédé selon la revendication 11, dans lequel ladite fonctionnalité choisie permet à un ou plusieurs polymères, dendrimères ou biomolécules d'être liés par un composé de formule (1).

13. Procédé selon la revendication 11 ou 12, dans lequel l'un des groupements R₁ ou R₂ peut être pourvu d'une fonctionnalité d'acide aminé de manière à faciliter la liaison d'une ou plusieurs biomolécules avec un substrat choisi.

14. Procédé selon la revendication 13, dans lequel un des groupements R₁ ou R₂ peut représenter le substituant suivant : 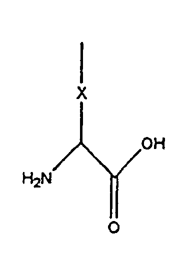 où X peut représenter un groupement contenant des hydrocarbures.

15. Procédé selon la revendication 14, dans lequel X représente un lieur d'alkylène -(CH₂)ₘ-, où m est 1 à 6, ou un lieur d'arylène -(CH₂)ₙ(p-C₆H₄)(CH₂)ₒ-, où n et o représentent indépendamment un nombre entier de 0 à 3.

16. Procédé selon l'une quelconque des revendications 2 à 4, dans lequel au moins l'un des groupements R₁ et R₂ comprend ladite substance biologique ou chimique choisie directement ou indirectement fixée au cycle de 1,3-dithiacyclopentane d'un composé de formule (1).

17. Procédé selon l'une quelconque des revendications 2 à 16, dans lequel R₃, R₄, R₅ et R₆ sont choisis dans le groupe constitué de l'hydrogène, des groupements fluoro, chloro, -R_{c}, -OR_{c}, -SR_{c} et -NR_{c}R_{d}, où R_{c} et R_{d} représentent un groupement alkyle en C₁₋₆ ou alcényle en C₂₋₆.

18. Procédé selon la revendication 17, dans lequel chacun des groupements R₃, R₄, R₅ et R₆ représente de l'hydrogène.

19. Procédé selon la revendication 17, dans lequel chacun des groupements R₃, R₄, R₅ et R₆ représente un halogène.

20. Procédé selon la revendication 19, dans lequel chacun des groupements R₃, R₄, R₅ et R-6 représente un groupement fluoro.

21. Procédé selon l'une quelconque des revendications 2 à 16, dans lequel R₃ et R₄ représentent conjointement =O et R₅ et R₆ représentent conjointement =O.

22. Procédé selon l'une quelconque des revendications 1 à 21, qui comprend par ailleurs l'application d'un second matériau sur au moins une surface du substrat.

23. Procédé selon la revendication 22, dans lequel le second matériau se présente sous la forme d'une SAM sélectivement formée dans des zones de la surface du substrat sensiblement non couvertes par une première SAM formée sur ladite surface selon l'une quelconque des revendications 1 à 21.

24. Procédé selon la revendication 23, dans lequel le 1,3-dithiacyclopentane de la première SAM est chimiquement distinct de la substance moléculaire de la seconde SAM.

25. Procédé selon la revendication 24, dans lequel la première SAM comprend une monocouche hydrophile et la seconde SAM comprend une monocouche hydrophobe.

26. Procédé selon la revendication 22, dans lequel le second matériau est sélectivement appliqué à des zones de la surface du substrat ressemblant sensiblement au motif de la première SAM formé sur ladite surface selon l'une quelconque des revendications 1 à 21.

27. Procédé selon la revendication 26, dans lequel le second matériau est un métal.

28. Procédé d'impression par microcontact, comprenant l'impression d'un motif sur une surface d'un substrat, dans lequel le motif comprend des régions exposées et des régions protégées par une SAM, la SAM étant formée par application sur au moins une surface du substrat d'un 1,3-dithiacyclopentane 2-mono- ou 2,2-disubstitué, le substituant en position 2 facilitant la formation de la SAM sur le substrat.

29. Procédé selon la revendication 28, dans lequel ledit 1,3-dithiacyclopentane est tel que défini dans l'une quelconque des revendications 1 à 21.

30. Procédé selon la revendication 28 ou 29, qui comprend l'application d'une estampe configurée définissant le motif requis de ladite couche configurée; et la mise en contact de ladite estampe configurée chargée d'encre avec la surface dudit substrat, ladite estampe configurée étant aménagée pour délivrer ladite encre aux zones de contact de la surface dudit substrat; ladite encre comprenant ledit 1,3-dithiacyclopentane 2-mono- ou 2,2-disubstitué.

31. Procédé selon la revendication 30, dans lequel l'estampe est formée de polydiméthylsiloxane.

32. Procédé selon l'une quelconque des revendications 1 à 31, dans lequel le substrat comprend un substrat métallique ou au moins une surface du substrat sur laquelle ladite SAM est formée comprend un métal.

33. Procédé selon la revendication 32, dans lequel le métal est de l'or.

34. Procédé d'impression par microcontact comprenant l'impression d'un motif sur une surface d'un substrat, le motif comprenant des régions exposées et des régions protégées par une SAM, la SAM étant formée par application sur au moins une surface du substrat du 1,3-dithiacyclopentane 2-monosubstitué suivant :

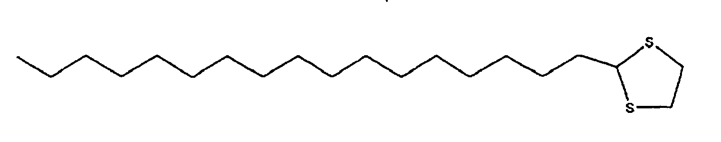

35. Procédé d'impression par microcontact, comprenant l'impression d'un motif sur une surface d'un substrat, où le motif comprend des régions exposées et des régions protégées par une SAM, la SAM étant formée par application sur au moins une surface du substrat du 1,3-dithiacyclopentane 2-monosubstitué suivant :

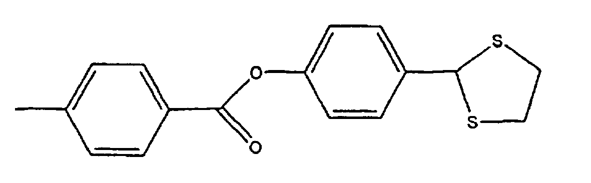

36. Composition d'encre pour usage en impression par microcontact, dans laquelle la composition comprend un 1,3-dithiacyclopentane 2-mono- ou 2,2-disubstitué, dans lequel le substituant en position 2 facilite la formation de la SAM sur un substrat conjointement avec un solvant convenant pour dissoudre le 1,3-dithiacyclopentane.

37. Composition d'encre selon la revendication 36, dans laquelle ledit 1,3-diathiacyclopentane 2-mono- ou 2,2-disubstitué se présente encore comme défini dans l'une quelconque des revendications 1 à 21.

38. Composition d'encre selon la revendication 36 ou 37, dans laquelle la concentration dudit 1,3-dithiacyclopentane dans ledit solvant est inférieure à 100 mM.

39. Composition d'encre selon la revendication 38, dans laquelle la concentration dudit 1,3-dithiacyclopentane dans ledit solvant se situe dans la plage d'environ 1,0 à 10,0 mM.

40. Composition d'encre selon l'une quelconque des revendications 36 à 39, dans laquelle ledit solvant est l'éthanol.

41. Composé de formule (Ic) : 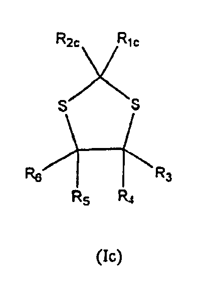 dans laquelle R_{1c} représente de l'hydrogène, R_{2c} représente un groupement alkyle en C₁₆₋₂₅ et R₃, R₄, R₅ et R₆ sont tels que définis dans l'une quelconque des revendications 2 et 17 à 21.

42. Composé selon la revendication 41, dans lequel R_{2c} représente un groupement heptadécyle et dans lequel R₃, R₄, R₅ et R₆ représentent de l'hydrogène.

43. Utilisation d'un 1,3-dithiacyclopentane 2-mono- ou 2,2-disubstitué, dans lequel la substitution en position 2 facilite la formation de la SAM sur un substrat à titre d'encre pour un usage en impression par microcontact.

44. Utilisation selon la revendication 43, dans laquelle ledit 1,3-dithiacyclopentane 2-mono- ou 2,2-disubstitué est tel que défini encore dans l'une quelconque des revendications 1 à 21.

45. Utilisation d'un 1,3-dithiacyclopentane 2-mono- ou 2,2-disubstitué, dans lequel le substituant en position 2 facilite la formation de la SAM sur un substrat, dans la fabrication d'une composition d'encre pour usage en impression par microcontact, ladite utilisation comprenant la dissolution dudit 1,3-dithiacyclopentane dans un solvant convenant au transfert dudit 1,3-dithiacyclopentane à une surface d'estampage.

46. Utilisation selon la revendication 45, dans laquelle ledit 1,3-dithiacyclopentane 2-mono- ou 2,2-disubstitué est tel que défini encore dans l'une quelconque des revendications 1 à 21.

47. Utilisation selon la revendication 45 ou 46, dans laquelle ledit solvant est l'éthanol.

48. Procédé de fabrication d'une composition d'encre pour usage en impression par microcontact, ledit usage comprenant la dissolution d'un 1,3-dithiacyclopentane 2-mono- ou 2,2-disubstitué, dans lequel le substituant en position 2 facilite la formation de la SAM sur un substrat, dans un solvant convenant au transfert dudit 1,3-dithiacyclopentane à une surface d'estampage.

49. Procédé selon la revendication 48, dans lequel ledit 1,3-dithiacycopentane 2-mono- ou 2,2-disubstitué est tel que défini encore dans l'une quelconque des revendications 1 à 21.

50. Procédé selon la revendication 48 ou 49, dans lequel ledit solvant est l'éthanol.

51. Trousse pour usage en impression par microcontact, ladite trousse comprenant une composition d'encre selon l'une quelconque des revendications 36 à 40; une estampe d'impression par microcontact pour transférer ledit 1,3-dithiacyclopentane de 2-mono- ou 2,2-disubstitué de ladite composition d'encre à un substrat; et un substrat convenant à la réception dudit 1,3-dithiacyclopentane 2-mono- ou 2,2-disubstitué de ladite composition d'encre à partir de ladite estampe.

52. Substrat configuré préparé selon un procédé conforme à l'une quelconque des revendications 1 à 35.

53. Substrat muni d'un motif sur au moins une de ses surfaces, où le motif comprend des régions exposées et des régions protégées par une SAM, où la SAM est formée par application à la surface d'un 1,3-dithiacyclopentane 2-mono- ou 2-disubstitué, le substituant en position 2 facilitant la formation de la SAM sur le substrat.

54. Substrat selon la revendication 53, dans lequel ledit 1,3-dithiacyclopentane 2-mono- ou 2,2-disubstitué est tel que défini encore dans l'une quelconque des revendications 1 à 21.

55. Utilisation d'un substrat selon l'une quelconque des revendications 52 à 54 à titre de résist de gravure.

56. Procédé de gravure d'un substrat, ledit procédé comprenant l'application d'une SAM sur un substrat selon l'une quelconque des revendications 1 à 35 et, ensuite, la mise en contact ensuite du substrat ainsi configuré avec une solution de gravure de manière à assurer une gravure dans les régions exposées du substrat non protégées par la SAM appliquée précédemment.

57. Utilisation d'un substrat selon l'une quelconque des revendications 52 à 54 dans l'immobilisation de matériaux chimiques et biologiques choisis sur celui-ci.

58. Utilisation de 1,3-dithiacyclopentane 2-mono- ou 2,2-disubstitué dans l'immobilisation de matériaux chimiques et biologiques choisis sur au moins une surface d'un substrat.

59. Utilisation selon la revendication 57 ou 58, dans laquelle ladite matière biologique est choisie dans le groupe constitué des peptides, des protéines, des acides oligo- et polynucléiques.

60. Utilisation selon la revendication 57 ou 58, dans laquelle ladite substance chimique est un polymère ou un dendrimère.
